# EUROPEAN PATENT SPECIFICATION

(11) **EP 1 494 746 B1**
(45) Date of publication and mention of the grant of the patent: **18.05.2011**
(21) Application number: 03723801.1
(22) Date of filing: 19.03.2003
(51) Int. Cl.: A61M 5/145

(54) **INSULIN FOR USE IN A DOSAGE REGIME**
INSULIN ZUR VERWENDUNG IN EINEM DOSIERVERFAHREN
INSULINE POUR L'UTILISATION DANS UN PROCEDE DE DOSAGE

(30) Priority: 29.03.2002 JP 2002094534; 26.12.2002 JP 2002377961
(43) Date of publication of application: 12.01.2005
(73) Proprietor: Aoki, Thomas, T., Sacramento, CA 95864 (US)
(72) Inventor: Aoki, Thomas, T., Sacramento, CA 95864 (US)
(74) Representative: Stainthorpe, Vanessa Juliet
(86) International application number: PCT/US2003/008781
(87) International publication number: WO 2003/084459

(56) References cited:
- US-A- 4 435 173
- US-A- 4 435 173
- US-A1- 2002 107 476
- US-A1- 2003 055 380
- US-A1- 2003 130 616

## Description

### FIELD OF INVENTION

The present invention relates to insulin for use in a method of treatment of a subject with impaired hepatic glucose processing.

### BACKGROUND OF THE INVENTION

Diabetic retinopathy is a major cause of blindness. While earlier detection and major advances in laser therapies have made significant impact on this chronic complication of diabetes, the number of diabetic patients suffering from diabetic retinopathy continues to increase.

Glucose control is typically measured by a blood test, which determines the level of hemoglobin A1c, which has been the desired result of insulin therapy in diabetic patients for many years. However, it is clear that tight circulating glucose control was insufficient in 25% or more of the study participants to protect them from the onset or progression of diabetic retinopathy, nephropathy or neuropathy.

A major cause of death for patients with diabetes mellitus is cardiovascular disease in its various forms. Existing evidence indicates that diabetic patients are particularly susceptible to heart failure, primarily in association with atherosclerosis of the coronary arteries and autonomic neuropathy. There is little doubt that a metabolic component is present in various forms of cardiovascular disease in diabetic patients. Cardiac dysfunction (lower stroke volume, cardiac index and ejection fraction and a higher left ventricular end diastolic pressure) frequently manifested by patients with diabetes, can be explained at least partially by metabolic abnormalities, and is likely secondary to insulin deficiency since appropriate insulin administration can restore normal patterns of cardiac metabolism (Avogaro et al, Am J Physiol 1990,258 : E606-18).

The pathophysiology of diabetic nephropathy is only partially understood. The most consistent morphologic finding in diabetic nephropathy is the enlargement of the mesangium, which can compress the glomerular capillaries and thus alter intraglomerular hemodynamics.

Diabetes is the number one cause of non-traumatic amputations. The common sources of amputations are wounds that will not heal and progress to necrosis and gangrene. It is generally observed that diabetic patients have greater difficulty in healing and in overcoming infections.

Diabetes in general and poor circulating glucose control in particular are thought to be causally related to poor wound repair in diabetic patients. Poor circulating glucose control is also a source of a lack of energy and a general feeling of malaise.

As reported in *Diabetes mellitus and the risk of dementia* A. Ott, RP. Stolk, F. Van Harskamp, The Rotterdam Study, Neurology, 1999, vol. 53, pp. 1937-1942, patients with diabetes have an increased risk of dementia. Having diabetes almost doubled the risk of having dementia (the risk was 1.9 times greater). The risk of diabetics getting Alzheimer's disease was also nearly double. And in diabetics taking insulin, the risk was over 4 times that in non-diabetics. Even after adjusting for possible effects of sex, age, educational level and the other factors measured, the findings were the same. Therefore, it can be concluded that diabetes is a risk factor for the development of dementias, including Alzheimer's disease.

What is is an insulin dosage regime by which: metabolism can be restored; retinal and neural glucose oxidation can be increased by enhancing pyruvate dehydrogenase activity; retinopathy and central nervous system disorders can be treated; stroke volume can be increased, that improves cardiac index; and ejection fraction can be increased, and that lowers ventricular end diastolic pressure, thus improving cardiac function, as well as improving the quality of life in diabetic patients. An insulin dosage regime is also needed to significantly reverse the cardiac dysfunction common to diabetic patients with heart disease. The same treatment should be capable of providing improved blood glucose control as measured by haemoglobin Alc. Additionally an insulin dosage regime is needed to improve the entire metabolic process and through its multiplicity of effects on neurovascular reactivity, intraglomerular pressure and hemodynamics, arrest the progression of overt diabetic nephropathy, improve intraglomerular hemodynamics, and thus arrest the progression of diabetic nephropathy and reduce the risk of development of End-Stage Renal Disease (ESRD). Further an insulin dosage regime is also needed to increase glucose oxidation in the affected areas and therefore provide more energy while consuming less oxygen for treating wounds, promote healing and avoid lower extremity amputations in both diabetic and non-diabetic patients. An insulin dosage regime is required to improve the metabolism in the brain of patients suffering with any of a number of diseases causing senile dementia and hence improve mental function of patients suffering senile dementia.

In a previous patent, US Patent No. 4,826, 810, the inventor describes a method of delivering pulses of insulin to a patient after ingestion of a glucose containing meal. The pulses of insulin are adjusted to produce a series of peaks in the free insulin concentration so that successively there are increasing free insulin concentration minima between the said peaks. In order to make this a viable treatment for clinical purposes there needs to be a simple, low-cost way of measuring free insulin to determine said peaks to insure that the correct levels are present to insure that the dietary carbohydrate processing capabilities of the subject's liver are activated. The only viable method for measuring "free" insulin is costly and time consuming, often taking days to obtain results. In the mean time it is not known whether or not the liver has been activated. What is needed is a way to determine, in real time while pulses are being administered and the base line of free insulin is rising, that in fact the patient's liver has been activated.

### SUMMARY OF THE INVENTION

The present invention is defined in claim 1.

The sub-claims define further features of the invention.

The infusion device delivers a series of pulses of insulin to the subject over a period of time accompanied by ingestion of glucose in the form of a carbohydrate containing meal. The amount of insulin in each pulse, the interval between pulses and the amount of time to deliver each pulse to the subject such as a patient are selected so that the hepatic processing of glucose is restored in subject. Initially after ingestion of the carbohydrate containing meal, the subject's circulating blood glucose level rises.

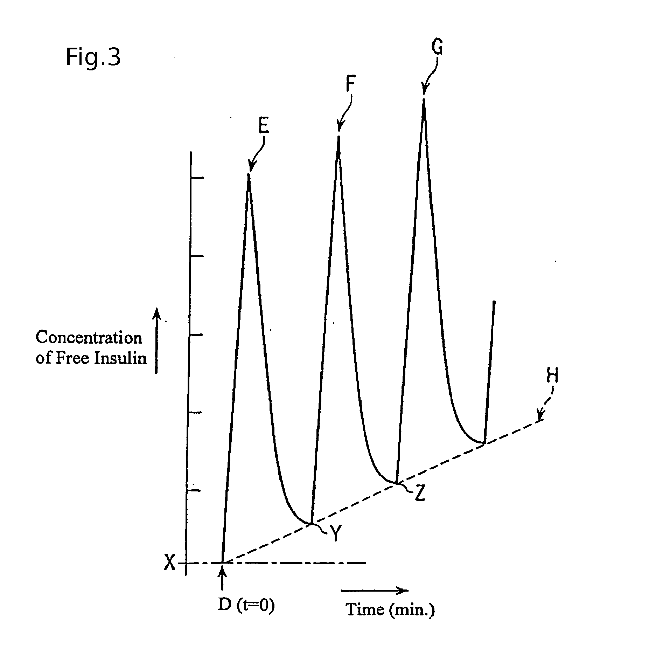

Coincident with or shortly following the establishment of elevated circulating glucose levels in the patient, the first pulse of insulin delivery is administered. This pulse results in a peak "free" insulin concentration in the blood represented by peak E (See FIG. 3). When the "free" insulin concentration decreases by about 90% to Y, the second pulse is administered, which results in peak F. When the "free" insulin concentration again decreases by about 90% to Z the next pulse is administered resulting in peak G. Repetition of this process will result in increasing interpeak "free" insulin concentration denoted by line H. The pulses are regulated so that the interpeak "free" insulin concentration increases by 10 to 500 µU/ml from one pulse to the next. In order to activate the liver, an increasing interpeak "free" insulin concentration after ingestion of a carbohydrate containing meal is required to activate the liver and for the circulating blood glucose level to drop 50 mg/dl in subjects with impaired hepatic glucose processing. However, there are times that even though the interpeak "free" insulin levels are rising, they do not rise sufficiently fast to activate the liver. In those circumstances the drop in circulating glucose will not reach 50 mg/dl.

It is desirable to administer the least amount of insulin consistent with activation of the hepatic glucose processing, yet the amount of insulin required to activate a patient will vary from patient to patient or even from day to day in the same patient. For the same patient on one day a pulse regimen will be successful in activation of hepatic glucose processing while the same patient on the following day requires significantly more insulin per pulse or more frequent pulses to attain activation. Measuring "free" insulin levels in the blood is an expensive and time-consuming procedure, which cannot provide the necessary information in real time. Disclosed in the current invention is a way to measure in real time when the patient has actually activated hepatic glucose processing to allow positive confirmation of successful patient response and signal when the pulses no longer need to be administered.

In subjects whose hepatic glucose processing has been restored there is a subsequent fall in circulating blood glucose levels of 50 mg/dl or more directly as a result of hepatic glucose processing being restored to the liver. This circulating glucose signal is easy and low cost to obtain, can be done by the patient easily in a home health care environment without the assistance of a doctor, and provides information in real time that the liver function is restored. Patients are usually well trained and fully capable of obtaining their own circulating glucose levels without the need of a doctor to assist with the procedure and evaluate the results. Other means to determine whether the liver has been activated are costly, do not provide information in real time, require a doctor's evaluation or cannot be used in a home health care environment. There must be more than a minimum of two pulses in the series of insulin pulses; for example, three, four, five or six. In the preferred embodiment the device is a pump which delivers a series of ten pulses over a period of one hour. The pump is preferably controlled by a programmable processor unit, which controls the amount of insulin in each pulse, the time to deliver each pulse, and the time between pulses. Circulating blood glucose levels can be measured by any appropriate circulating glucose measuring method including finger stick methods.

### DRAWINGS

Herein follows a description of the drawings:
FIG. 1 is a schematic block diagram of a programmable insulin pump utilizing a syringe plunger-type of mechanism and programmed to deliver insulin;
FIG. 2 is a schematic block diagram of a programmable insulin pump with a rotating plunger mechanism and programmed to deliver insulin according to the present invention; and
FIG. 3 is a graph showing the relation between the concentration of insulin in the blood and time in a series of insulin pulses.

### DESCRIPTION OF PREFERRED EMBODIMENTS

Accordingly, the present invention includes an infusion device for administering insulin to diabetic patients by infusing a series of pulses of insulin into the patient at regular intervals. At the same time the patient ingests a carbohydrate containing meal and circulating glucose measurements are made periodically to insure proper hepatic processing of glucose has been restored.

Liquid or food containing glucose is consumed by the patient to prevent the patient from becoming hypoglycemic. The preferred liquid or food containing glucose is 4 to 10 ounces of GLUCOLA for diabetic patients which translates to 40 to 100 grams of glucose, but any similar type of liquid or high glycemic food, including but not limited to cake and bread, containing glucose may be given to the patient. For non diabetic patients ingested glucose amounts are higher and need be adjusted to each patient.

In the preferred embodiment of the invention, a programmable insulin pump is programmed to deliver intravenous insulin in precisely measured pulses, programmed to deliver each of those pulses within a minimum amount of time, and programmed to allow for timed intervals between each pulse. However, any method of infusing measured amounts of insulin to include simple injection with a syringe is also acceptable. The preferred means of insulin delivery is a programmable infusion device capable of providing measured pulses of insulin on a prearranged interval, so long as there is sufficient glucose in the blood to keep the patient from becoming hypoglycemic. It is also preferable that the infusion device is capable of delivering the pulses of insulin in as short duration of time as possible, without adversely affecting the vein at the site of infusion is used. One preferred infusion device is the Bionica MD-110. However, less accurate devices and slower devices, to include a simple syringe, may deliver the pulses and achieve the needed infusion profile.

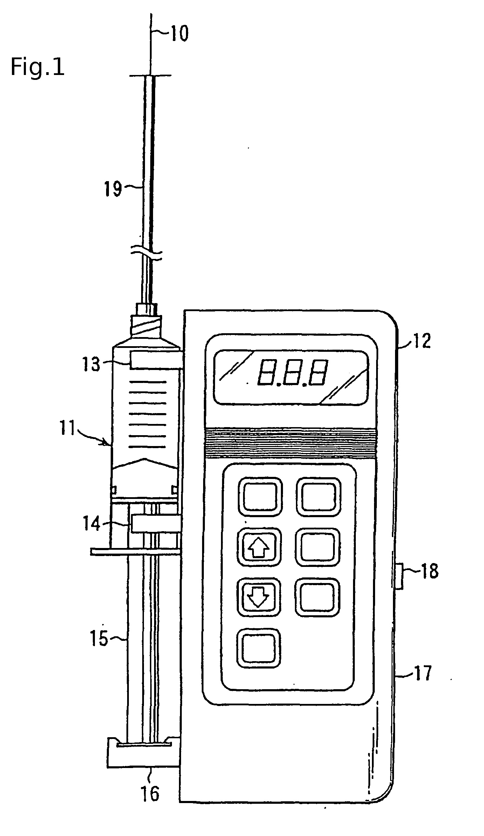

In one embodiment a programmable, handheld pump uses a conventional medical syringe for infusing the insulin. Referring to FIG. 1 the syringe 11 is attached to the pump by clips 13 & 14 and the plunger 15 is activated by the syringe driver 16. The syringe driver is actuator driven by any of a number of possible actuator configurations known to those skilled in the art. Any conventional infusion tube connection device may be used to connect the infusion tube to the syringe. Programmed values can be input to a control processor via the keyboard 17, through firmware in the pump or by software via a communications link 18 to a higher level computer or any other appropriate input method. A circulating glucose measuring instrument, configured to communicate directly with the pump through the communications link can provide timely values of circulating glucose. Alternatively, wireless communications systems can send information from a circulating glucose sensor automatically to the pump without operator intervention. Typical circulating glucose sensors include but are not limited to finger stick devices, non-invasive instruments using near infrared spectroscopy or radio frequency, and implanted sensors. Alternatively the circulating glucose signal can come from an implantable system for monitoring pancreatic beta cell electrical activity in a patient in order to obtain a measure of a patient's insulin demand and circulating glucose level. Any other method for either directly or indirectly obtaining an accurate measure of the change in circulating glucose levels is also acceptable. The communications link may be used to send alarm and status messages to a higher level computer via any acceptable communications protocol and medium.

When the pump is activated, it dispenses the programmed pulse of insulin in the programmed amount of time to the subject. The insulin travels through the infusion tube 19, to the needle 10, which is inserted intravenously into the subject wherever convenient but preferably in the forearm. The time to deliver each pulse should be as short as possible and at least less than one minute and preferably on the order of seconds. Pump status, alarm status and circulating-glucose levels, among other parameters of the system may be displayed on the display panel 12.

In the preferred embodiment the subject's circulating glucose levels are measured periodically and used either automatically or manually to input changes to parameters of the pump delivering the insulin. Adjustments to ingested glucose and infused insulin are then made to produce the desired results of activating the liver without the unwanted side effects of either hypoglycemia or hyperglycemia.

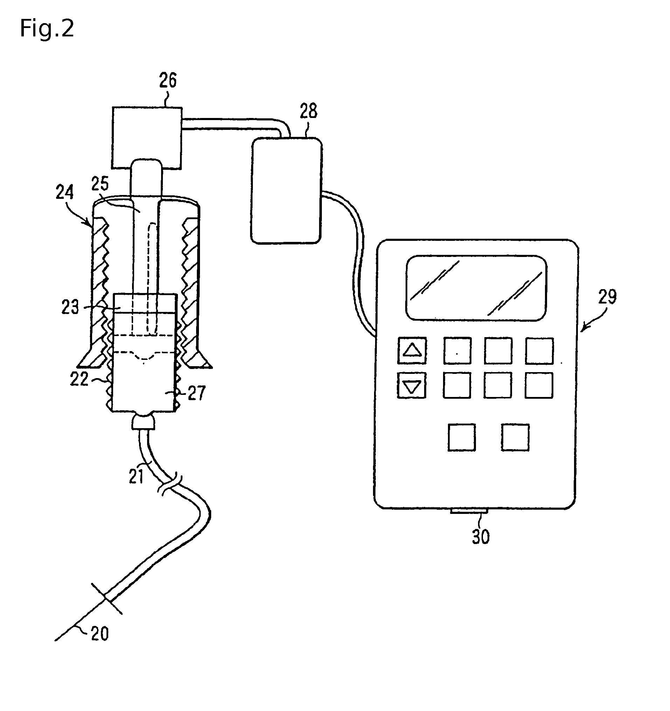

In another embodiment instead of a syringe type pump, referring to FIG. 2, the infusion pump comprises a cassette cartridge pump device, consisting of a cartridge 22, a housing 24, a plunger 25, a reservoir area 27 within the cartridge where the insulin is contained, and a neck opening for the connection of the cartridge to an infusion tube 21 to an infusion needle 20, which is inserted intravenously into the subject wherever convenient but preferably in the forearm. The pumping mechanism comprises a gear linkage 26, a motor 28 and a pumping device 29. When the cartridge 22 is engaged in the housing 24, the cartridge is locked into place by the rotational engagement of outside threads of the cartridge and inside threads of the housing. The cartridge is made optionally of glass, ceramic, steel or plastic. The pumping mechanism is used to rotate the piston-type plunger 25 and 23 within the cassette cartridge. The pumping mechanism may be actuated by methods including but not limited to any motor which rotates the plunger or housing, by a coordinated hand-eye movement or by manual movement through a series of "click" points. The plunger rotates in relation to the walls of the cassette housing. As the plunger 25 is rotationally turned, the device infuses insulin to the subject. Preferably the pumping mechanism is controlled by a programmable processor which controls the amount of insulin to be infused, the timing between pulses and the length of time to infuse a single pulse. The diameter of the reservoir area and the depth the plunger travels for each plunger rotation are selected to provide highly accurate pulse sizes delivered in as short a time as is safely possible. Programmed values can be input to a control processor via the keyboard, through firmware in the pump or by software via a communications link 30 from a higher level computer or any other appropriate input method. Automated entry of blood glucose levels can be accomplished as described above. The same communications link may be used to send alarm and status messages to a higher level computer via any acceptable communications protocol and medium. Pump status, alarm status and circulating-glucose levels, among other parameters of the system may be displayed on the display panel of the pumping device 29.

Hepatic processing of glucose includes proper uptake of glucose in the liver cells, oxidation of glucose by the liver cells, storage of glucose as hepatic glycogen in the liver cells, and conversion of glucose to fat or alanine, an amino acid, by the liver cells. Hepatic processing is impaired when the liver fails to produce hepatic enzymes (specifically hepatic glucokinase, phosphofructokinase, and pyruvate kinase) needed in proper glucose processing. Impaired processing of glucose is a fundamental condition of type 1 and type 2 diabetic patients, for patients whose pancreas is not producing sufficient insulin, and for patients experiencing significant insulin resistance, or a combination of these factors. After the ingestion of glucose, even with intravenous insulin administration, decreased glucose oxidation, low alanine production, and little glycogen formation and deposition in the liver in a timely manner are all indications that hepatic glucose processing is impaired. Glucose tolerance tests and measurements of hemoglobin Alc can be used as indications that hepatic processing of glucose has been impaired.

In one preferred embodiment, insulin pulses are delivered to a patient utilizing Chronic Intermittent Intravenous Insulin Therapy, as follows. On the morning of the procedure, the patient is preferably seated in a blood drawing chair and a 23 gauge needle or catheter is preferably inserted into a hand or forearm vein to obtain vascular access. However, any system of such access may accomplish the needed result, including indwelling catheters, PICC lines and PortaCaths. After a short equilibration period, the patient is asked to make a circulating glucose measurement prior to starting the actual infusion of insulin. It is preferable that patients have circulating glucose levels close to 200 mg/dl prior to using the infusion device. In the case of pregnant diabetic women, however, every attempt is made to keep the maximum circulating glucose level to 180 mg/dl or less.

After the circulating glucose measurement has been taken and the patient has the proper circulating glucose starting level, the patient is asked to consume a liquid or food containing glucose. The amount of glucose given to the diabetic patient ranges from 60 to 100 grams or 6 to 10 ounces of GLUCOLA, but for small framed people the amount could be as low as 40 grams of glucose or 4 ounces of GLUCOLA. However, the amount of initial glucose given to the patient may vary. In the non-diabetic patient more glucose may be required than in the diabetic patient, but the other parameters would remain the same, including the need for a pulsed delivery. Pulses of insulin are then administered intravenously at planned intervals of time, usually every six minutes however other intervals may be used from as low as every three minutes up to every 30 minutes. For diabetic patients the amount of insulin in each pulse is 10-200 milliunits of insulin per kilogram of body weight; for non-diabetic patients slightly lower.

Circulating glucose measurements are made as frequently as possible. When finger pricks are used, because of the discomfort to the patient, it is recommended that readings be taken every 30 minutes. When less invasive methods of measuring circulating glucose are used readings can be taken more frequently, preferably after the infusion of each pulse of insulin. It is recommended that a period of one to two minutes is allowed after the infusion of each pulse of glucose before circulating glucose levels are measured. The circulating glucose level will typically rise by approximately 100 to 150 mg/dl before starting to fall. In patients whose hepatic glucose processing has been restored there will be the fall in circulating glucose levels by about 50-100 mg/dl. In patients who have yet to obtain proper hepatic glucose processing, there will be no fall or a fall considerably less than 50 mg/dl. The fall in circulating glucose levels, indicating restoration of hepatic processing of glucose, is generally achieved within one hour of initiation of the first pulse of insulin using the preferred embodiment of this invention; however, the time required may be shorter or longer than one hour. It is possible to decrease the amount of insulin in each pulse and to lengthen the time between pulses so that it takes in excess of two or even three hours or more for a fall of 50 mg/dl to occur. The longer the time it takes to activate the patient, however, the longer the patient must be under treatment and the less desirable the treatment is for the patient. This decrease in circulating glucose is caused by the combination of increased glucose utilization by muscles and the use of glucose by the liver.

Another indication that hepatic activation of the liver has been reestablished is that gradually the amount of insulin required to reduce the circulating glucose levels by 50 mg/dl or more will decrease with time. Lowering hemoglobin A1 c levels are a more mid-term manifestation that hepatic processing has been restored. Longer-term manifestations are seen in the decrease of a number of complications related to diabetes, including but not limited to retinopathy, nephropathy, neuropathy, hypoglycemia, cardiovascular disease, and hypertension.

The phase during which a series of pulses of insulin is administered and glucose ingested lasts typically for 56 minutes (ten pulses with a six minute interval between pulses) and is followed by a rest period of usually one or two hours. The rest period allows the elevated insulin levels to return to baseline. During periods when insulin is not being infused, the intravenous site is preferably converted to a heparin or saline lock. The entire procedure is repeated until the desired effect is obtained. Typically the procedure is repeated three times for each treatment day, but can be repeated as few as two times and up to 8 times in one day. Prior to the patient being discharged from the procedure, whether in the clinic or home environment, in the preferred embodiment circulating glucose levels stabilize at 100-200 mg/dl for approximately 30-45 minutes.

Accordingly, the present invention provides an insulin dosage regime to increase retinal and neural glucose oxidation by enhancing pyruvate dehydrogenase activity and therefore treats retinopathy and central nervous system disorders in both diabetic and non-diabetic patients. One method of monitoring retinal and neural glucose oxidation is PET (Positron Emission Tomography) scans. Alternatively, one may look for stabilization/reversal of diabetic retinopathy. In terms of neural function, there will be improvement in peripheral neuropathy manifested as increased perception of sensation, especially in the feet, and a loss of the painful "burning" or "pins and needles "sensation in the feet. There will also be improvement in autonomic neuropathy, especially gastroparesis and improvement in postural or orthostatic hypotension.

Diabetic heart disease is the one of the more common complications of diabetes, experienced by both type I and type II diabetic patients. Experts generally agree that the primary fuel for both the normal and diabetic heart is free fatty acids, a fuel that requires more oxygen on a per calorie basis than glucose as a fuel. As a consequence, the heart of both diabetic and nondiabetic individuals is particularly vulnerable to ischemia. If the involved tissue had been primarily utilizing free fatty acids for energy generation, even a slight or temporary decrease in blood flow or oxygen supply would be catastrophic. On the other hand, if that tissue had been oxidizing glucose rather than free fatty acids, for the generation of an equivalent amount of energy, a temporary disruption of blood or oxygen supply would not be as deleterious, since that tissue's oxygen requirements would be less. Thus, for the same amount of oxygen delivered to the myocardium, glucose utilization rather than free fatty acid utilization would result in increased energy (ATP) generation. The present invention is capable of improving the dietary fuel processing capabilities by allowing for more glucose to be burned or oxidized and correcting over utilization of free fatty acids associated with heart disease and cardiovascular disease in both diabetic and non-diabetic patients.

Still further, the present invention is capable of improving the entire metabolic process, and, through its multiplicity of effects on neurovascular reactivity, intraglomerular pressure and hemodynamics, of arresting the progression of overt diabetic nephropathy, of improving intraglomerular hemodynamics, thus arresting the progression of diabetic nephropathy, and reducing the risk of development of ESRD in both diabetic and non-diabetic patients.

Further, the present invention is capable of increasing glucose oxidation in an affected area and thereby providing more energy with the same oxygen delivery for treating wounds, promoting healing and avoiding amputations in both diabetic and non-diabetic patients. The rationale for this improved healing is that the tissue surrounding the affected area suffers from inadequate blood supply, leading to insufficient oxygenation. When this tissue is fuelled through enhanced glucose oxidation in lieu of free fatty acid utilization, thereby switching from a predominantly lipid based fuel economy to one based more on glucose oxidation, more energy is available for wound healing for the same amount of blood flow and hence, more healing from the amount of oxygen delivered. In addition, the ability to achieve more energy from less oxygen, thereby addresses a general malaise associated with diabetic individuals who have energy levels which are less than normal.

On many occasions patients who have been diabetics as well as having dementia have been treated with insulin pulses. Dementia appears to be related to poor metabolism of glucose in the brain, which may well be the result of constricted flow of blood. This poor metabolism is at least in part the cause of the dementia. Use of the treatment regime according to the current invention in patients suffering from senile dementia has clearly shown improvement in confusion, weakness, disorientation, cognitive function and lack of memory associated with dementia as well as improvement in the blood glucose management. Constricted flow of blood to the brain is also prevalent in demented patients without diabetes and the device of the current invention provides improved metabolism as well to those patients and hence is effective in treating both demented patients with and without diabetes.

In the preferred embodiment, with a new patient two successive days of three treatments are performed the first week. For continuing patients the procedure is performed once a week. For patients who need/require a more intensive approach, the procedure may be repeated 3 or more times, including continuously, each week until the desired clinical outcome is achieved.

The following non-limiting examples are given by way of illustration only.

### EXAMPLE1

A study was conducted to assess the effects of Chronic Intermittent Intravenous Insulin Therapy (CIIIT) on the progression of diabetic nephropathy in patients with type 1 diabetes mellitus (DM). This 18-month multi-center, prospective, controlled study involved 49 type 1 DM patients with nephropathy who were following the Diabetes Control and Complications Trial (DCCT) intensive therapy (IT) regimen. Of these, 26 patients formed the control group C, which continued on IT, while 23 patients formed the treatment group (T) and underwent, in addition to IT, weekly CIIIT. All study patients were seen in clinic weekly for 18 months, had monthly glycohemoglobin HbA1c checked, and every 3-months urinary protein excretion and creatinine clearance (CrCl) determinations. CrCl declined significantly in both groups as expected, but the rate of CrCl decline in the T group (2.21± 1.62 ml/min/yr) was significantly less than in the C group (7.69 ± 1.88 ml/min/yr, P=0.0343). The conclusion is that when CIIIT is added to IT in type 1 DM patients with overt nephropathy, it appears to markedly reduce the progression of diabetic nephropathy.

### EXAMPLE 2

A middle-aged woman with Type 1 diabetes for more than 22 years suffered from polyneuropathy. She had generalized pain and was unable to walk or even wear stockings because of the pain. After receiving treatment with the subject device the pain has been reduced to the point where the woman enjoys rigorous exercise such as roller blading.

### EXAMPLE 3

A middle-aged woman with Type 1 diabetes for more than 30 years had severe peripheral neuropathy, was in constant pain below the knees and had difficulty sleeping at night. After receiving treatment with the subject device, she no longer takes pain medication and has no twinges of pain in her legs. She has been using the treatment for eight years.

### EXAMPLE 4

A middle-aged woman with type 2 diabetes for 17 years was suffering from severe dilated cardiomyopathy (ejection fraction 14-19%). She was placed on the list to receive a heart transplant prior to starting treatment with the subject device. After receiving treatment, the subject reduced her insulin intake from 150 units a day to 24-26 units/day, and she stabilized to the point where she no longer required a heart transplant and, indeed, was removed from the heart transplant list. The patient has been receiving treatment for 9 years and is still off the heart transplant list. Her ejection fraction is currently 29-32%.

### EXAMPLE 5

A middle-aged male with type 1 diabetes for 38 years suffered from macular degeneration (retinopathy). He was unable to drive at night. After receiving treatment with the subject device, the man's eyesight improved to the point where night driving was no longer a concern. The patient has been receiving treatment for 4 years.

### EXAMPLE 6

A middle-aged type 2 diabetic male patient had severe heart disease including congestive heart failure and severe artereosclerotic heart disease. The patient was scheduled for heart surgery but because of his poor condition, surgeons refused to operate. After using the subject device, the doctors were convinced that he could withstand 4-vessel by-pass surgery. The patient had a normal postoperative recovery, which is virtually unheard of for diabetic patients with his stage of heart disease.

### EXAMPLE 7

An older type 2 diabetic male patient was exercising and had excellent circulating glucose control under intense insulin therapy including 3-4 injections per day of subcutaneous insulin. Even so, his diabetes related kidney disease had progressed to the point where he was discharging 1500 milligrams of protein during a 24-hour period and the rate of increase was 500 milligrams/24 hours/year. After using the subject device, the patient's proteinuria was reduced to 600-800 milligrams/24 hours. He has been using the device for 5 years.

### EXAMPLE 8

An older type 1 diabetic female patient who was diabetic from age 5 years old was scheduled for a coronary artery by-pass graft to correct her diabetes related heart disease. The surgeons were reluctant to operate in the condition she was in because of her advanced diabetes related arteriosclerosis. She was scheduled for a single vessel graft. After using the subject device, her condition improved to the point where the doctors performed two instead of one grafts. She had a normal recovery. She continuing using the subject device for several years after the surgery with no further deterioration in her diabetes related heart disease.

### EXAMPLE 9

An older type 2 diabetic male suffering with autonomic neuropathy had very elevated blood pressure readings of 200/120 despite a rigorous program to regulate his circulating glucose using intensive insulin therapy of 3 to 4 subcutaneous insulin injections daily. As a result of using the subject device, his blood pressure decreased to 120/80. He has been using the device for 5 years.

### EXAMPLE 10

An older type 2 diabetic male patient had one amputated leg as a result of diabetes related ulcers on that leg. He had developed ulcers on the other leg that would not respond to any available therapy and was in danger of losing the other leg to amputation. As a result of using the subject device, the ulcers on his second leg healed, and the leg was saved from amputation. This patient used the subject device for several more years, and no additional ulcers formed.

### EXAMPLE 11

A middle-aged type 1 female diabetic patient had developed severe ulcers on both legs, which would not heal with any available treatment. As a result of using the subject device, the ulcers healed and have never returned. The patient has been using the subject device now for 13 years.

### EXAMPLE 12

A middle-aged type 2 male diabetic patient had proliferative diabetic retinopathy with severe bleeding. Multiple photocoagulation scars made additional photocoagulation impossible. As a result of using the subject device the bleeding stopped, and there was no further deterioration of the retina, preserving what eyesight he had left. The patient has been using the subject device for 5 years, and he has had no further bleeding of the retina and no further photocoagulation.

### EXAMPLE 13

An elder type 2 female diabetic patient had severe painful peripheral neuropathy to the point that she was unable to walk and used a wheelchair. After six months of using the subject device, the pain had subsided to the point where she no longer used a wheelchair. Because of financial reasons, she stopped the therapy. As a result, the neuropathy returned, and she returned to using a wheelchair.

### EXAMPLE 14

A middle-aged type I female diabetic patient had severe neuropathy. She was a mother of two children who was bed-ridden with autonomic neuropathy before using the subject device two years ago. Her muscles had atrophied, she could not digest her food, she had been told that her nerves were dying inside her as a result of her diabetes. She stated that if she had not have two children, she would have taken her life. She had to quit her job, went on disability and was in an out of the hospital very often. She had welts on her head causing hair loss. She had no sensation in her feet, she had constant nausea, and she couldn't sleep at night because of the pain. She had insulin absorption problems and tried all different ways to improve the absorption of insulin into her body. For a number of years she injected herself intramuscularly because she felt that she obtained the best absorption of insulin that way. Since using the subject device she has reversed all of the diseases to the point where she has taken herself off disability and is gainfully employed. She has not been in the hospital since. The numbness in her legs has gone away. If she skips the treatment for a week, she can feel the numbness return to her legs. Her gastroparesis was reversed, and she no longer suffers symptoms. Aside from using the subject device she has no medical costs now.

### EXAMPLE 15:

A 79 year old female diabetic who was suffering from advanced senile dementia was placed in a nursing home because of excessive confusion, weakness, disorientation and lack of memory. Because the nursing home was not keeping up the strict four shot regimen needed by the patient for her diabetic blood sugar control, the patient's children removed the patient from the nursing home. The attending doctor recommended Hepatic Activation. Once the patient was activated, she returned totally to an independent living style. She had significant improvement in her motor skills, memory, and cognitive function. Hepatic Activation clearly had a positive effect on her senile dementia.

For all of the above listed examples, after the initial few days of treatment, the patients underwent treatment once a week, each treatment day consisting of three infusions of insulin accompanied by ingestion of carbohydrates. The pump device used to infuse the insulin was the Bionica MD-110 pump described in FIG. 1 without the sensor for circulating glucose. Typically there were ten pulses given over a period of one hour, and a rest period of one hour was taken between infusions of insulin. The form in which the carbohydrates were ingested changed from time to time and included eating foods of high glycemic index including but not limited to bread and cake. The patients' circulating glucose was measured once every thirty minutes by the finger stick method currently used by most diabetic patients. Circulating glucose levels initially rose during each treatment and then fell between 50 and 100 mg/dl during each series of insulin infusions indicating that in fact the liver had been activated. Table 1 below summarizes by the above examples the number of units of insulin per pulse administered and the amount of glucose ingested for each series of pulses:
The preferred embodiments described herein are illustrative only, and although the examples given include many specificity's, they are intended as illustrative of only a few possible embodiments of the invention. Other embodiments and modifications will, no doubt, occur to those skilled in the art. The examples given should only be interpreted as illustrations of some of the preferred embodiments of the invention, and the full scope of the invention should be determined by the appended claims

**TABLE 1**

| Summary of the above examples: The number of units of insulin per pulse administered and the amount of glucose ingested for each series of pulses | | |
|---|---|---|
| Example Number | Number of milliunits of insulin/Kg of body weight per Pulse | Grams of Glucose per Series of Insulin Pulses. |
| 1 * | 15-195 | 40-100 grams |
| 2 | 30-45 | 50-60 grams |
| 3 | 35-50 | 40-60 grams |
| 4 | 45-60 | 40-60 grams |
| 5 | 30-45 | 50-60 grams |
| 6 | 70-100 | 50-70 grams |
| 7 | 40-60 | 50-70 grams |
| 8 | 15-45 | 50-70 grams |
| 9 | 40-55 | 50-70 grams |
| 10 | 45-60 | 40-60 grams |
| 11 | 15-45 | 50-70 grams |
| 12 | 130-170 | 50-70 grams |
| 13 | 30-60 | 50-70 grams |
| 14 | 30-60 | 50-70 grams |
| 15 | 30-60 | 50-70 grams |

| | | |
|---|---|---|
| * This study included 23 patients in the treatment group with varying amounts of insulin per pulse and varying ingestion of glucose. Hence general limits of what they used are included. | | |

## Claims

1. Insulin for use in a method of treatment of a subject with impaired hepatic glucose processing, said method comprising the steps of:
the ingestion by the subject of a carbohydrate containing meal;
delivery to the subject of a series of pulses of said insulin by an infusion device,
wherein the amount of said insulin in each pulse, the interval between pulses and the amount of time to deliver each pulse to the subject are selected so that the circulating glucose level in the subject, as monitored by a monitoring device, initially rises and then falls by an amount equal to 50 mg/dl or more.

2. Insulin according to claim 1, wherein the treatment is to improve energy levels of the subject.

3. Insulin according to claim 1, wherein the treatment is to improve one or more of confusion, weakness, disorientation, cognitive function or lack of memory in patients suffering dementia.

4. Insulin according to claim 1, wherein the treatment is to arrest the progression of nephropathy reducing the risk of end stage renal disease.

5. Insulin according to claim 1, wherein the treatment is to correct over utilisation of fatty acids associated with heart disease and cardiovascular disease.

6. Insulin according to claim 1, wherein the treatment is to stabilize or reverse the progression of retinopathy.

7. Insulin according to claim 1, wherein the treatment is to improve the symptoms of autonomic neuropathy including gastroparesis and orthostatic hypotension.

8. Insulin according to claim 1, wherein the treatment is to improve or arrest the progression of peripheral neuropathy.

9. Insulin according to claim 1, wherein the treatment is to decrease hypertension and/or to decrease the amount of anti-hypertension medication required.

10. Insulin according to claim 1, wherein the treatment is of wounds to promote healing and avoid amputations.

11. Insulin according to any of claims 1 to 10, wherein said infusion device comprises a cartridge (22) containing liquid insulin and a plunger (15;25), and wherein the plunger is rotatable to dispense the insulin.

12. Insulin according to any of claims 1 to 10 wherein said infusion device comprises a syringe (11) to deliver pulses of insulin.

13. Insulin according to any of claims 1 to 10 wherein the falling of the circulating glucose level in the subject by an amount equal to 50mg/dl or more occurs within two hours of administering the first pulse of the series of pulses.

14. Insulin according to any of claims 1 to 10, wherein the pulses of insulin are administered intravenously.

15. Insulin according to any of claims 1 to 10 wherein the amount of insulin in each pulse is between 10 and 200 milliunits per kilogram of body weight.

16. Insulin according to any of claims 1 to 10 wherein said infusion device comprises a programmable processor unit and the amount of insulin in each pulse, the time interval between pulses and the amount of time to deliver each pulse to the subject are controlled by said programmable processor unit.

17. Insulin according to any of claims 1 to 10 wherein one said series of pulses is delivered over a period of 6 to 180 minutes.

18. Insulin according to any of claims 1 to 10 wherein each pulse of the said series of pulses is delivered every 3 to 30 minutes.

19. Insulin according to any of claims 1 to 10 wherein said carbohydrate containing meal contains between 40 to 100 grams of glucose.

20. Insulin according to any of claims 1 to 10 wherein said delivery of said series of pulses is performed from 2 to 8 times a day.

21. Insulin according to any of claims 1 to 10 where information is transferred automatically between a blood glucose meter and a pump.

22. Insulin according to any preceding claim wherein said subject suffers from diabetes.

## Patentansprüche

1. Insulin zum Einsatz in einem Verfahren zur Behandlung eines Patienten mit gestörter hepatischer Glucoseverarbeitung, wobei das genannte Verfahren die folgenden Schritte umfasst:
die Aufnahme einer kohlenhydrathaltigen Mahlzeit durch den Patienten;
die Verabreichung einer Reihe von Impulsen des genannten Insulins durch eine Infusionsvorrichtung an den Patienten;
wobei die Menge des genannten Insulins in jedem Impuls, das Intervall zwischen Impulsen und die Dauer der Verabreichung jedes Impulses an den Patienten so ausgewählt werden, dass der zirkulierende Glucosewert in dem Patienten gemäß der Überwachung durch eine Überwachungsvorrichtung anfänglich ansteigt und danach um eine Höhe von 50 mg/dl oder mehr sinkt.

2. Insulin nach Anspruch 1, wobei die Behandlung dazu dient, die Energiewerte des Patienten zu verbessern.

3. Insulin nach Anspruch 1, wobei die Behandlung der Verbesserung einer oder mehrerer der folgenden Zustände dient: Konfusion, Schwäche, Orientierungslosigkeit, kognitive Funktion oder Erinnerungslosigkeit bei unter Demenz leidenden Patienten.

4. Insulin nach Anspruch 1, wobei die Behandlung dem Anhalten des Fortschreitens von Nephropathie dient, wobei die Gefahr einer terminalen Niereninsuffizienz reduziert wird.

5. Insulin nach Anspruch 1, wobei die Behandlung der Korrektur der Überverwertung von Fettsäuren in Verbindung mit einer Herzerkrankung und einer Herz- und Gefäßkrankheit dient.

6. Insulin nach Anspruch 1, wobei die Behandlung der Stabilisierung oder Umkehr des Fortschreitens von Retinopathie dient.

7. Insulin nach Anspruch 1, wobei die Behandlung der Verbesserung der Symptome der autonomen Neuropathie dient, einschließlich Gastroparese und orthostatischer Hyotension.

8. Insulin nach Anspruch 1, wobei die Behandlung der Verbesserung oder des Anhaltens des Fortschreitens der peripheren Neuropathie dient.

9. Insulin nach Anspruch 1, wobei die Behandlung der Verringerung der Hypertonie und/oder der Verringerung der benötigten Menge eines Arzneimittels zur Behandlung von Hypertonie dient.

10. Insulin nach Anspruch 1, wobei Wunden behandelt werden, um deren Heilung zu fördern und um Amputationen zu verhindern.

11. Insulin nach einem der Ansprüche 1 bis 10, wobei die genannte Infusionsvorrichtung eine Patrone (22), welche Insulin in flüssiger Form enthält, und einen Kolben (15, 25) umfasst, und wobei der Kolben zur Abgabe des Insulins drehbar ist.

12. Insulin nach einem der Ansprüche 1 bis 10, wobei die genannte Infusionsvorrichtung eine Spritze (11) zur Verabreichung von Insulinimpulsen umfasst.

13. Insulin nach einem der Ansprüche 1 bis 10, wobei das Sinken des zirkulierenden Glucosewertes in dem Patienten um eine Menge von 50 mg/dl oder mehr innerhalb von zwei Stunden nach Verabreichung des ersten Impulses der Reihe von Impulsen erfolgt.

14. Insulin nach einem der Ansprüche 1 bis 10, wobei die Insulinimpulse intravenös verabreicht werden.

15. Insulin nach einem der Ansprüche 1 bis 10, wobei die Menge des Insulins in jedem Impuls zwischen 10 und 200 Millieinheiten je Kilogramm Körpergewicht liegt.

16. Insulin nach einem der Ansprüche 1 bis 10, wobei die genannte Infusionsvorrichtung eine programmierbare Prozessoreinheit umfasst, und wobei die Insulinmenge in jedem Impuls, das Zeitintervall zwischen Impulsen und die Dauer der Verabreichung jedes Impulses an den Patienten durch die genannte programmierbare Prozessoreinheit gesteuert werden.

17. Insulin nach einem der Ansprüche 1 bis 10, wobei eine genannte Reihe von Impulsen über einen Zeitraum von 6 bis 180 Minuten verabreicht wird.

18. Insulin nach einem der Ansprüche 1 bis 10, wobei jeder Impuls der genannten Reihe von Impulsen alle 3 bis 30 Minuten verabreicht wird.

19. Insulin nach einem der Ansprüche 1 bis 10, wobei die genannte kohlenhydrathaltige Mahlzeit zwischen 40 und 100 Gramm Glucose enthält.

20. Insulin nach einem der Ansprüche 1 bis 10, wobei die genannte Verabreichung der genannten Reihe von Impulsen zwischen zwei- und achtmal täglich vorgenommen wird.

21. Insulin nach einem der Ansprüche 1 bis 10, wobei Informationen automatisch zwischen einer Blutglucosemessvorrichtung und einer Pumpe übertragen werden.

22. Insulin nach einem der vorstehenden Ansprüche, wobei der genannte Patient an Diabetes leidet.

## Revendications

1. Insuline à utiliser dans un procédé de traitement d'un sujet avec une transformation du glucose hépatique altérée, ledit procédé comprenant les étapes consistant à :
ingérer, pour le sujet, un repas contenant des glucides ;
délivrer au sujet une série d'impulsions de ladite insuline par un dispositif de perfusion,
dans lequel la quantité de ladite insuline dans chaque impulsion, l'intervalle entre les impulsions et le temps pour délivrer chaque impulsion au sujet sont choisis de sorte que le taux de glucose circulant dans le sujet, tel que contrôlé par un dispositif de contrôle, commence par monter puis diminue d'une valeur égale à 50 mg/dl ou plus.

2. Insuline selon la revendication 1, dans laquelle le traitement vise à améliorer les niveaux d'énergie du sujet.

3. Insuline selon la revendication 1, dans laquelle le traitement vise à améliorer un ou plusieurs de la confusion, la faiblesse, la désorientation, la fonction cognitive ou le manque de mémoire chez les patients souffrant de démence.

4. Insuline selon la revendication 1, dans laquelle le traitement vise à arrêter la progression d'une néphropathie réduisant le risque de maladie rénale en phase terminale.

5. Insuline selon la revendication 1, dans laquelle le traitement vise à corriger une utilisation excessive d'acides gras associée à une maladie cardiaque ou à une maladie cardiovasculaire.

6. Insuline selon la revendication 1, dans laquelle le traitement vise à stabiliser ou inverser la progression d'une rétinopathie.

7. Insuline selon la revendication 1, dans laquelle le traitement vise à améliorer les symptômes d'une neuropathie autonome y compris la gastroparésie et l'hypotension orthostatique.

8. Insuline selon la revendication 1, dans laquelle le traitement vise à améliorer ou arrêter la progression d'une neuropathie périphérique.

9. Insuline selon la revendication 1, dans laquelle le traitement vise à diminuer une hypertension et/ou à diminuer la quantité de médicaments anti-hypertension nécessaires.

10. Insuline selon la revendication 1, dans laquelle le traitement concerne les plaies afin de favoriser la guérison et éviter les amputations.

11. Insuline selon l'une quelconque des revendications 1 à 10, dans laquelle ledit dispositif de perfusion comprend une cartouche (22) contenant l'insuline liquide et un piston (15 ; 25), et dans laquelle le piston peut tourner pour dispenser l'insuline.

12. Insuline selon l'une quelconque des revendications 1 à 10, dans laquelle ledit dispositif de perfusion comprend une seringue (11) pour délivrer des impulsions d'insuline.

13. Insuline selon l'une quelconque des revendications 1 à 10, dans laquelle la chute du taux de glucose circulant dans le sujet d'une valeur égale à 50 mg/dl ou plus survient dans les deux heures suivant l'administration de la première impulsion de la série d'impulsions.

14. Insuline selon l'une quelconque des revendications 1 à 10, dans laquelle les impulsions d'insuline sont administrées par voie intraveineuse.

15. Insuline selon l'une quelconque des revendications 1 à 10, dans laquelle la quantité d'insuline dans chaque impulsion est comprise entre 10 et 200 milli-unités par kilogramme de poids corporel.

16. Insuline selon l'une quelconque des revendications 1 à 10, dans laquelle ledit dispositif de perfusion comprend une unité à processeur programmable et la quantité d'insuline dans chaque impulsion, l'intervalle de temps entre les impulsions et le temps pour délivrer chaque impulsion au sujet sont contrôlés par ladite unité à processeur programmable.

17. Insuline selon l'une quelconque des revendications 1 à 10, dans laquelle l'une desdites séries d'impulsions est délivrée sur une période de 6 à 180 minutes.

18. Insuline selon l'une quelconque des revendications 1 à 10, dans laquelle chaque impulsion desdites séries d'impulsions est délivrée toutes les 3 à 30 minutes.

19. Insuline selon l'une quelconque des revendications 1 à 10, dans laquelle ledit repas contenant des glucides contient entre 40 et 100 grammes de glucose.

20. Insuline selon l'une quelconque des revendications 1 à 10, dans laquelle la délivrance desdites séries d'impulsions est effectuée de 2 à 8 fois par jour.

21. Insuline selon l'une quelconque des revendications 1 à 10, dans laquelle les informations sont automatiquement transférées entre un glucomètre et une pompe.

22. Insuline selon l'une quelconque des revendications précédentes, dans laquelle ledit sujet souffre de diabète.
